# FASCICULE DE BREVET EUROPEEN

(11) **EP 4 670 118 B1**
(45) Date de publication et mention de la délivrance du brevet: **07.10.2026**
(21) Numéro de dépôt: 24712114.8
(22) Date de dépôt: 21.02.2024
(51) Int. Cl.: G06T 7/00, G01N 23/046, G01N 33/00

(54) **PROCÉDÉ DE CONTRÔLE NON-DESTRUCTIF DE PIÈCES EN MATÉRIAU COMPOSITE**
VERFAHREN ZUR ZERSTÖRUNGSFREIEN PRÜFUNG VON VERBUNDWERKSTOFFTEILEN
METHOD FOR NON-DESTRUCTIVE TESTING OF PARTS MADE OF COMPOSITE MATERIAL

(30) Priorité: 21.02.2023 FR 2301591
(43) Date de publication de la demande: 31.12.2025
(73) Titulaire: Safran, 75015 Paris (FR)
(72) Inventeur: BELKHIR, Nacim, 77550 MOISSY-CRAMAYEL (FR); MENDOZA QUISPE, Arturo, 77550 MOISSY-CRAMAYEL (FR)
(74) Mandataire: Regimbeau
(86) Numéro de dépôt international: PCT/FR2024/050229
(87) Numéro de publication internationale: WO 2024/175861

(56) Documents cités:
- EP-A1- 3 054 286
- WO-A1-2007/071788
- FR-A1- 3 050 826
- FR-A1- 3 055 729
- HRISHIKESH BALE ET AL: "Characterizing Three-Dimensional Textile Ceramic Composites Using Synchrotron X-Ray Micro-Computed-Tomography", JOURNAL OF THE AMERICAN CERAMIC SOCIETY, vol. 95, no. 1, 31 January 2012 (2012-01-31), US, pages 392 - 402, XP055331937, ISSN: 0002-7820, DOI: 10.1111/j.1551-2916.2011.04802.x

## Description

### DOMAINE TECHNIQUE

La présente invention concerne le domaine du contrôle non destructif (CND) de pièces en matériau composite, notamment, dans le domaine de l'aéronautique, d'aubes en composite tissé. Plus particulièrement, elle concerne le contrôle de ce type de pièces à l'aide d'images tomographiques de celles-ci.

### ETAT DE LA TECHNIQUE

Les matériaux composites, sont communément utilisés du fait de leur résistance et de leur légèreté. Typiquement, un tel matériau composite comporte un renfort, par exemple en fibres de carbone, et une matrice de résine dans laquelle ledit renfort est noyé. Dans le domaine aéronautique notamment, où la sécurité et la fiabilité sont des préoccupations majeures, les pièces obtenues doivent faire l'objet de différents types d'analyses et de contrôles pour vérifier leur bonne réalisation et l'absence de défauts.

En effet, selon la localisation des défauts dans le matériau, en effet, leur proximité ou leur nature (changement de taux volumique de fibre, modification du ratio chaines/trames), la détection de la présence de défauts peut remettre en cause l'intégrité ou la fiabilité de la pièce.

Une méthode de contrôle non destructif (CND) classiquement utilisée consiste à acquérir des images tomographiques des pièces à contrôler et à faire analyser ces images par des experts. Ceux-ci vont généralement inspecter une multitude de coupes dimensionnelles de la pièce, ces coupes étant choisies pour balayer la pièce selon ses trois dimensions.

On comprend que ce type de contrôle « manuel » n'est pas satisfaisant. Il est fastidieux et peut prendre plusieurs heures du fait de la taille importante des pièces. En outre, il est fortement lié à l'expertise métier de l'opérateur : les variabilités pour un même-opérateur ou entre opérateurs peuvent porter préjudice à la qualité du contrôle.

Il existe ainsi un besoin de fiabiliser la qualité du CND, en s'affranchissant au maximum de ces variabilités, pour améliorer la répétabilité, et en augmenter le rendement.

Le document FR 3 050 826 A1 propose la mise en œuvre d'une technique Apprentissage semi-supervisée basée sur l'approche dite « Mean teacher » afin de décharger l'opérateur de cette étape de contrôle « manuel » des pièces en matériau composite.

Cependant, la faible quantité de données annotées par des opérateurs qualifiés et le défaut de qualité des données disponibles, entre autres, ne permettent pas une utilisation satisfaisante d'une telle technique d'apprentissage. De plus, la technique proposée dans ce document ne réalise qu'un classement selon un type binaire, attribuant soit une valeur « sans défaut » (« sain ») soit une valeur « défaut » (« anomalie »). Or un tel classement ne permet pas d'obtenir une détection fiable qui minimise les fausses alarmes (c'est-à-dire des zones saines détectées comme défectueuses). En effet, certaines zones présentant un défaut pourront être classées comme « sans défauts » ou bien de nombreuses zones « sans défauts » pourront être classées comme avec « défaut ». En outre, elle ne présente pas de résultats robustes vis-à-vis de la détection de défauts de tissage nouveaux ou bien jamais observés préalablement et/ou enregistrés.

On connait par ailleurs d'autres techniques d'apprentissage. En particulier, la méthode d'apprentissage non-supervisée permet de classer des données non étiquetées en identifiant des similarités. Cependant, la qualité des images disponibles ainsi que la grande similarité entre les propriétés des matériaux sains et ceux défectueux ne permettent pas de mise en œuvre satisfaisante de ces techniques en particulier dans le domaine de l'aéronautique, d'aubes en composite tissé.

D'autres documents de l'état de la technique pertinents sont WO 2007/071788 A1, EP 3 054 286 A1 et Hrishikesh Bale et al.: "Characterizing Three-Dimensional Textile Ceramic Composites Using Synchrotron X-Ray Micro-Computed-Tomography", Journal of the American Ceramic Society, vol. 95, no. 1, 31 janvier 2012, pages 392-402.

### EXPOSE GENERAL

Un but général de l'invention est de proposer un procédé permettant de détecter, dans un tissage, et au moyen d'un nombre de données limitées, un ensemble de défauts ou d'éventuels défauts, de manière rapide et robuste.

Il est à cet effet proposé, selon un aspect de la présente invention, un procédé de contrôle non destructif d'une pièce composite tissée dont un volume tomographique a été préalablement acquis et divisé en zones de visualisations, caractérisé en ce qu'il comprend les étapes suivantes de :
- pré-traitement dudit volume tomographique, comprenant un découpage des zones de visualisations du volume tomographique en des sous-volumes tridimensionnels ;
- traitement de chaque sous-volume, ledit traitement comprenant, pour chaque sous-volume, d'une part, une extraction d'un vecteur descripteur, ladite extraction mettant en œuvre une analyse d'image au moyen d'un modèle de représentation préalablement appris, ladite analyse d'image décomposant chaque sous-volume en une pondération appliquée à une pluralité de descripteurs dudit modèle de représentation, le vecteur descripteur étant composé des poids de cette décomposition, et d'autre part, une projection du vecteur descripteur extrait dans un modèle de réduction de dimension préalablement appris, de manière à condenser le vecteur descripteur en un vecteur descripteur réduit, de dimension plus faible ;
- post-traitement des vecteurs descripteurs réduits, comprenant : d'une part une conversion, au moyen d'un modèle d'estimation de vraisemblance préalablement appris, du vecteur descripteur réduit de chaque sous-volume en une valeur scalaire, ladite valeur scalaire représentant une vraisemblance d'appartenance du sous-volume à une classe saine, et d'autre part une agrégation par calcul de moyenne des valeurs scalaires dans chacune des zones de visualisation, le résultat de ce calcul étant une estimation de l'appartenance à la classe saine pour chaque zone de visualisation du volume tomographique ; et
- contrôle d'une présence de défauts sur la pièce composite tissée, en fonction des estimations de l'appartenance à la classe saine des zones de visualisation du volume tomographique ainsi obtenues.

Ce procédé combine, entre autres, la création d'un modèle de représentation et d'un modèle d'estimation de vraisemblance. Cette combinaison permet la détection et la localisation d'un ensemble d'anomalies, y compris celles qui n'ont jamais été observées, ou bien, qui sont indisponibles dans la base d'apprentissage.

De plus, ce procédé propose des résultats de localisation plus robustes que l'art antérieur en réduisant le nombre de « faux positifs », autrement dit de zones faussement catégorisées comme saines.

En outre, ce procédé améliore le contrôle en le simplifiant et le rendant plus rapide.

Avantageusement, mais facultativement, le procédé exposé comprend l'une au moins des caractéristiques suivantes, prise seule ou dans une quelconque combinaison :
- une étape de création du modèle de représentation, permet l'extraction de vecteurs descripteurs, et comprenant des étapes de : sélection de sous-volumes de référence, formant un volume tomographique de référence, annotés comme étant d'un type sain ou d'un type défectueux, diversification des sous-volumes de référence, ladite diversification mettant en œuvre une multiplication et une transformation géométrique d'un ou plusieurs sous-volumes de référence, et apprentissage d'un réseau de neurones au moyen des sous-volumes de référence diversifiés pour obtenir un modèle de représentation des sous-volumes de référence, l'apprentissage mettant en œuvre une fonction d'erreur et un algorithme d'optimisation. Ceci permet, entre autres, d'augmenter la représentation des sous-volumes comprenant un défaut et de réaliser le contrôle non destructif malgré le manque de sous-volumes annotés ;
- une étape de création du modèle de réduction de dimension propre à permettre la projection de vecteurs descripteurs extraits, et comprenant la mise en œuvre : de l'extraction de vecteurs descripteurs de référence des sous-volumes de référence, au moyen du modèle de représentation, d'un calcul de construction d'un espace réduit, ledit calcul de construction mettant en œuvre une orthogonalisation d'une première matrice de covariance des vecteurs descripteurs de référence extraits. Ceci permet, entre autres, de condenser les vecteurs descripteurs et donc de minimiser l'effet dit de la malédiction de dimension » ;
- une étape de création du modèle d'estimation de vraisemblance, propre à permettre la conversion de vecteurs descripteurs réduits, ladite création mettant en œuvre : la projection, dans l'espace réduit, des vecteurs descripteurs de référence extraits annotés comme étant type sain, de manière à les condenser en des vecteurs descripteurs de référence réduits de type sain, et un calcul d'une seconde matrice de covariance des vecteurs descripteurs de référence réduits de type sain représentant un domaine de la classe saine. Ceci permet, entre autres, de mesurer correctement une distance entre la valeur scalaire et le domaine de la classe saine et donc la normalité ;
- la projection du vecteur descripteur en un vecteur descripteur réduit met en œuvre une méthode d'analyse en composantes principales. Ceci permet, entre autres, de projeter chaque nouveau vecteur descripteur dans l'espace réduit directement et sans nécessiter d'étapes supplémentaires ;
- la conversion du vecteur descripteur réduit de chaque sous-volume en valeur scalaire met en œuvre un calcul d'une distance de Mahalanobis. Ceci permet, entre autres, de minimiser l'influence des composantes les plus bruitées ;
- l'apprentissage d'un réseau de neurones met en œuvre une approche semi-supervisée dite « Mean Teacher » utilisant deux réseaux de neurones dont le poids de l'un est défini comme une moyenne mobile exponentielle du poids de l'autre. Ceci permet, entre autres, d'obtenir un vecteur descripteur de dimension 2048 et non une réponse binaire ;
- l'apprentissage du réseau de neurones met en œuvre la fonction d'erreur de type « Focal Loss » ou de type entropie croisée. Ceci permet, entre autres, de pénaliser de mauvaises prédictions et plus particulièrement pour des jeux de données mal équilibrés ;
- la création du modèle de représentation comprend un regroupement des sous-volumes de référence obtenus par la diversification en des sous-ensembles comprenant chacun des sous-volumes de référence de types sain et défectueux. Ceci permet, entre autres, de faire en sorte que le réseau ne surapprenne pas sur des sous-volumes de types sains ;
- la création du modèle de représentation comprend un échantillonnage aléatoire répartissant uniformément dans le volume tomographique les sous-volumes de référence sélectionnés et annotés de type sain. Ceci permet, entre autres, de rééquilibrer les sous-volumes sélectionnés selon leur type ;
- le pré-traitement comprend une étape initiale de correction mettant en œuvre un réglage du format et du taux de compression du volume tomographique ;
- le pré-traitement comprend une étape de mise à plat du volume tomographique de manière à réaliser le traitement sur un volume exempt d'une courbure indépendante d'une orientation locale du tissage ;
- le pré-traitement comprend une étape de normalisation globale des niveaux de gris du volume tomographique adapté pour en améliorer le contraste ;
- le découpage des zones de visualisations consiste en un pavage en sous-volumes de tailles 100x100xD voxels, D étant une dimension de l'épaisseur du volume tomographique. Ceci permet, entre autres, d'améliorer le traitement statistique mis en œuvre par la suite ;
- le découpage des zones de visualisations met en œuvre un recouvrement de 50% entre chaque paire de sous-volume adjacent, d'une même zone de visualisation uniquement, afin de minimiser le taux de fuite. Ceci permet, entre autres, d'éliminer les biais dans le cas où un défaut serait situé entre deux sous-volumes ;
- le pré-traitement comprend une étape de correction locale du contraste mettant en œuvre une normalisation d'histogramme de chaque sous-volume découpé ;
- l'étape de correction locale du contraste est mise en œuvre par un algorithme de type CLAHE (soit « contrast limited adaptive histogram equalization » selon la terminologie anglo-saxonne). Ceci permet, entre autres, d'améliorer le contraste local tout en limitant l'amplification du bruit ;
- le contrôle d'une présence de défauts sur la pièce composite tissée met en œuvre une comparaison des estimations de l'appartenance à la classe saine à un seuil prédéfini.

Selon un autre aspect, il est proposé un produit programme d'ordinateur, comprenant des instructions de code pour exécuter le procédé de contrôle non destructif lorsqu'il est exécuté sur des moyens de traitement d'une unité de calcul.

### DESCRIPTION DES FIGURES

D'autres caractéristiques, buts et avantages ressortiront de la description qui suit, qui est purement illustrative et non limitative, et qui doit être lue en regard des dessins annexés sur lesquels :
les **figures 1a, 1b** et **1c** illustrent des exemples de défauts de tissages ;
la **figure 2** illustre des étapes d'un procédé de contrôle non destructif d'une pièce composite tissée selon une mise en œuvre de la présente invention ;
la **figure 3** illustre un exemple d'une vue de pièce composite tissé sur laquelle sont reportées des zones de visualisation ;
la **figure 4** illustre un découpage d'une zone de visualisation volume tomographique en sous-volumes selon une mise en œuvre de la présente invention ;
la **figure 5** illustre des étapes d'un apprentissage d'un modèle de détection selon une mise en œuvre de la présente invention ;
la **figure 6** illustre schématique des étapes d'un procédé d'analyse de la qualité de pièces.

Sur l'ensemble des figures, les éléments similaires portent des références identiques.

### DESCRIPTION DETAILLEE

### Généralités

Le procédé de contrôle non destructif (CND) mis en œuvre peut être appliqué à différentes étapes de fabrication de la pièce composite tissée **1** à contrôler, par exemple une aube de soufflante ou encore de turbine. Selon la pièce, le matériau composite peut être différent (résine à matrice de carbone, résine à matrice céramique, etc...).

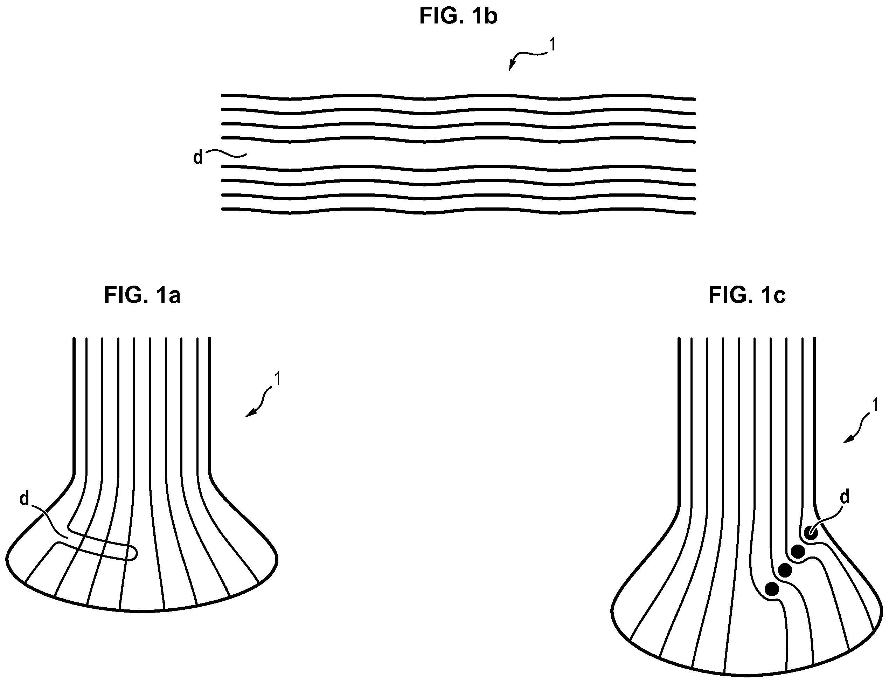

Le contrôle non-destructif de la pièce **1** a pour finalité la détection de défauts de tissage ou d'injection **d** dans la pièce, typiquement :
- une boucle : un toron de carbone, de chaine ou de trame, ne suit pas sa trajectoire théorique et forme une boucle à l'intérieur de la pièce **(****figure 1a****) ;**
- un manque : un toron de carbone, de chaine ou de trame, est absent **(****figure 1b****).**
un flambage, avec ou sans accumulation de résine **(****figure 1c****).**

### Procédé de contrôle non-destructif

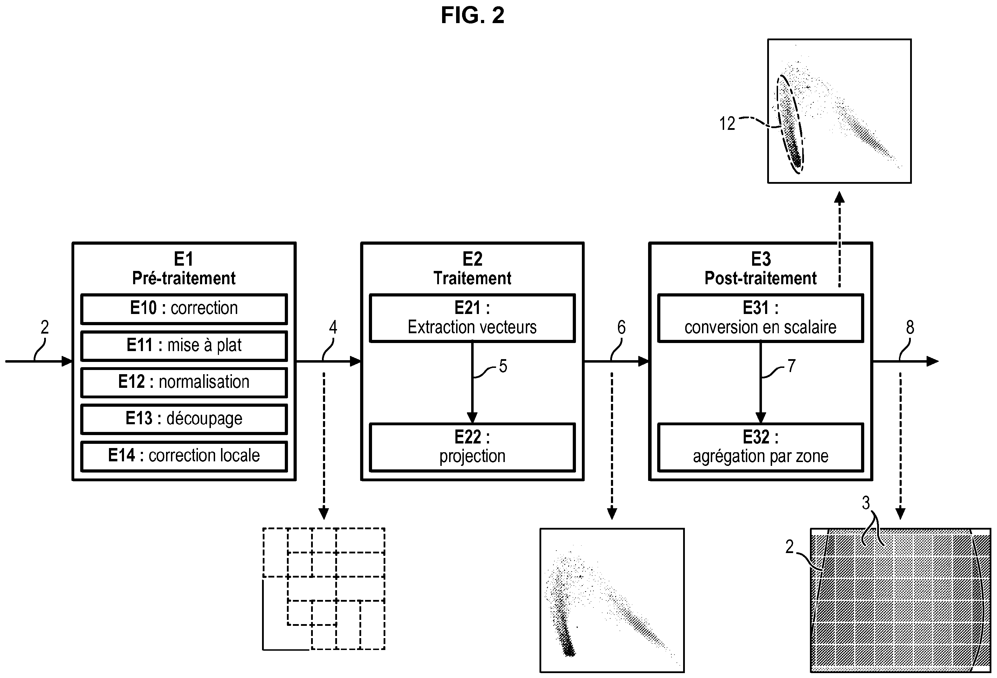

La **figure 2** illustre des étapes du procédé de contrôle non destructif d'une pièce composite tissée **1.** Une image 3D de la pièce (volume tomographique) est préalablement acquise par reconstruction à partir d'une pluralité de coupes 2D de la pièce, obtenues par exemple par radiographie sous rayons X.

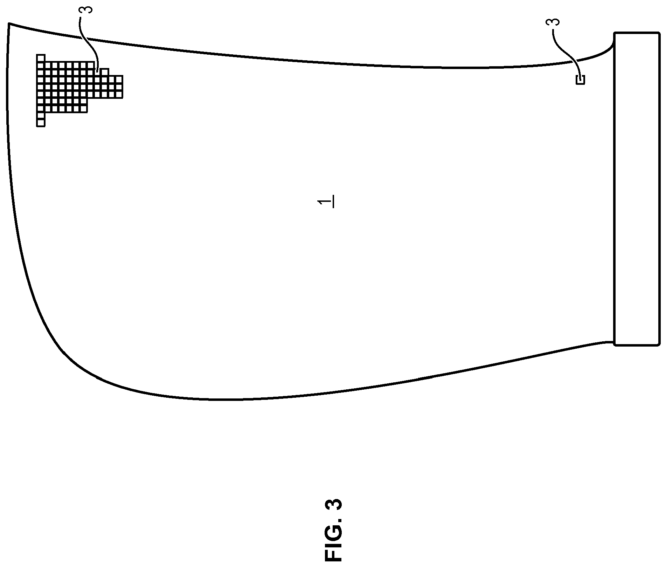

Comme illustré schématiquement, par exemple, sur la **figure 3****,** ce volume tomographique **2** acquis de la pièce composite tissée **1** est préalablement divisé en une pluralité de zones de visualisation **3** (volumes) typiquement quelques dizaines de zones de visualisations **3,** par exemple 60 zones de visualisations 3, présentant chacune une taille de 250x250XD voxels, avec D représentant l'épaisseur (en nombre de voxels) de la pièce représentée par le volume tomographique **2.**

Chaque zone de visualisation **3** de la pièce tissée est tout d'abord soumise à un pré-traitement **E1** permettant d'obtenir un découpage en sous-volumes **4** tridimensionnels.

Les sous-volumes **4** sont ensuite soumis à un traitement **E2** qui permet d'obtenir, pour chaque sous-volume **4,** un vecteur descripteur réduit **6.**

Les vecteurs descripteurs réduits **6** sont eux-mêmes soumis à un post-traitement **E3** pour obtenir une estimation de l'appartenance **8** à la classe saine pour chaque zone de visualisation **3** du volume tomographique **2.**

Enfin, le procédé comprend un contrôle **E4** prenant en compte l'estimation de l'appartenance **8** à la classe saine pour chaque zone de visualisation **3.**

### Ces différentes étapes E1 à E4 sont détaillées ci-après

L'étape de pré-traitement **E1** comprend elle-même plusieurs sous-étapes. Tout d'abord, le volume tomographique **2** est corrigé (étape **E10)** afin de permettre sa lecture. La correction **E10** comprend un réglage du format et du taux de compression du volume tomographique **2.** Cette étape peut être suivie d'une mise à plat **E11** du volume tomographique **2** tridimensionnel. La mise à plat met en œuvre la suppression des courbures du volume tomographique 2 qui ne sont pas liées au tissage en lui-même. Autrement dit, le volume tomographique **2** est projeté tridimensionnellement sur un plan afin d'en supprimer la courbure qui est indépendante des orientations locales du tissage, qui n'est donc pas considéré comme un défaut d de tissage mais comme une contrainte fixée et recherchée lors de la conception. Cette étape permet de ne conserver que les courbures liées aux orientations locales du tissages.

En outre, le pré-traitement **E1** comprend une normalisation globale des niveaux de gris **E12** du volume tomographique **2.** Cette normalisation **E12** met en œuvre une uniformisation pour atténuer des artéfacts d'acquisition, de sorte que le niveau de gris de chaque voxel du volume tomographique **2** soit proportionnel à la densité de matière de la pièce en ce point. La normalisation **E12** élimine les valeurs extrêmes de la distribution des niveaux de gris pour augmenter le contraste global du volume tomographique **2.** Les valeurs extrêmes sont par exemple définies par les 5% percentiles de chaque extrémité de la distribution, en d'autres termes les 5% des valeurs les plus élevées et les plus faibles. La normalisation **E12** permet ainsi de fiabiliser les étapes ultérieures.

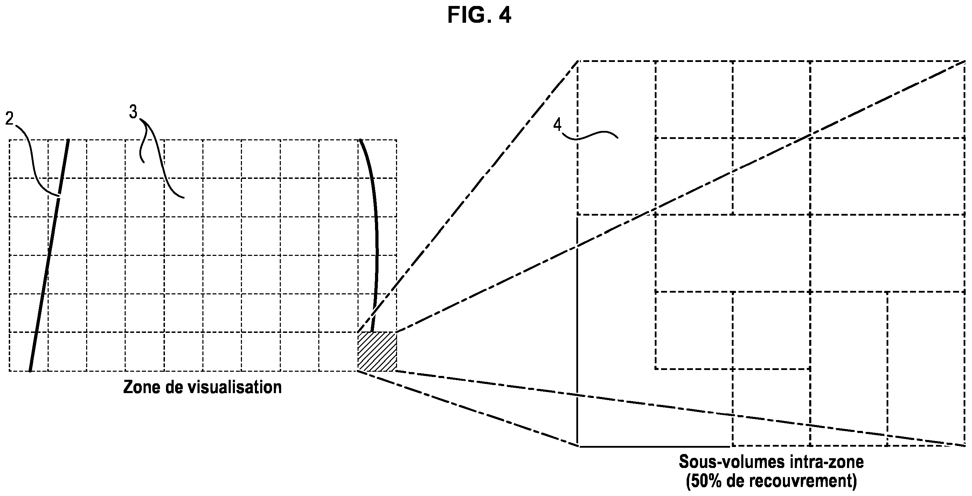

Les zones de visualisation du volume tomographique **2** sont découpées (étape **E13)** au cours du pré-traitement **E1** comme cela est illustré par la **figure 4****.** Le découpage **E13** réalise un pavage de chaque zone de visualisation **3** du volume tomographique **2** en sous-volumes **4** élémentaires tridimensionnels. Les sous-volumes **4** présentent par exemple une taille de 100x100xD voxels, de manière à correspondre à une taille représentative d'un motif de tissage (l'unité élémentaire du tissage d'un point de vue matériau/mécanique). A cette fin, chaque sous-volume **4** doit vérifier deux conditions : d'une part être plus grand que la taille caractéristique des constituants (ici les torons, par exemple) afin que leur nombre soit suffisant pour permettre une représentation statistique correcte du volume étudié, et d'autre part, être plus petit que la taille caractéristique des zones de visualisation **3,** afin de pouvoir appliquer un traitement statistique dans chacune de ces zones de visualisation **3.** De plus, la détermination du sous-volume **4** dépend des motifs de tissages utilisés dans la pièce composite. Le sous-volume **4** est préférablement orienté selon les directions de la pièce **1.**

L'étape de découpage **E13** réalise un recouvrement entre chaque paire de sous-volume **4** adjacent, d'une même zone de visualisation **3** uniquement, afin de minimiser le taux de fuite. Le taux de recouvrement entre chaque paire adjacente de sous-volume **4** peut être de 20% à 80% et préférentiellement de 50%. Un tel recouvrement de 50% permet de faire en sorte que chaque voxel du volume tomographique **2** soit compris dans deux sous-volumes **4.** Le recouvrement vise à éliminer des erreurs en cas de présence d'un défaut **d** entre deux sous-volumes **4.** De plus, ce découpage **E13** se restreint à un recouvrement des sous-volumes **4** « intra-zones de visualisation **3** ». Ainsi le découpage **E13** ne réalise pas de recouvrement des sous-volumes **4** de zones de visualisation **3** distinctes. Cela permet, dans la suite du procédé, que les vecteurs descripteurs réduits **6** de chaque sous-volume **4** portent sur une portion du volume tomographique **2** totalement comprise dans une seule zone de visualisation **3.** Ainsi les estimations de l'appartenance **8** à la classe saine pour chaque zone de visualisation **3** peuvent être agrégés par zone de visualisation **3,** par une simple opération statistique, sans nécessiter le développement d'un algorithme dédié à la répartition des estimations de l'appartenance **8.**

Le découpage est suivi d'une étape de correction locale **E14** du contraste de chaque sous-volume **4.** La correction locale **E14** met en œuvre un algorithme de normalisation d'histogramme de type CLAHE (« Contrast Limited Adaptive Histogram Equalization » selon la terminologie anglo-saxonne). De plus, la correction locale **E14** met en œuvre une simple normalisation de « blanchissement » des sous-volumes **4** pour qu'ils aient une moyenne nulle et une variance unitaire. Cette correction locale **E14** est plus robuste qu'une correction globale et améliore ainsi la fiabilité du traitement ultérieur. De plus la méthode CLAHE permet de limite l'amplification du bruit créé par la normalisation des histogrammes.

Suit l'étape de traitement **E2** qui est réalisée sur chacun des sous-volumes **4** prétraité et comprend plusieurs étapes successives. Tout d'abord, le traitement met en œuvre une extraction **E21** d'un vecteur, dit vecteur descripteur **5,** de chaque sous-volume **4.** L'extraction **E21** est une analyse d'image au moyen d'un modèle de représentation **9** préalablement appris. Le modèle de représentation **9** est obtenu par un apprentissage dont un mode de mise en œuvre est détaillé ci-après. L'analyse d'image décompose chaque sous-volume **4** en une pondération appliquée à une pluralité de descripteurs du modèle de représentation. Les poids de ces descripteurs composent ainsi le vecteur descripteur **5.**

Les vecteurs descripteurs **5** extraits de chaque sous-volume **4,** par l'extraction **E21,** sont ensuite soumis à une projection **E22.** Cette étape met en œuvre un modèle de réduction **10** préalablement appris qui permet de condenser chaque vecteur descripteur **5** extrait d'un sous-volume **4** en un vecteur descripteur réduit **6.** A cette fin, la projection **E22** met en œuvre une méthode paramétrique comme par exemple une analyse en composantes principales (ou « Principal Component Analysis », selon la terminologie anglo-saxonne). Cette analyse permet d'obtenir les dimensions principales de chaque vecteur descripteur **5** par une simple projection dans un espace réduit obtenu grâce au modèle de réduction **10** sans nécessiter d'étapes supplémentaires. Les dimensions principales du vecteur descripteur **5** synthétisent les informations importantes et forment le vecteur descripteur réduit **6.** La dimension du vecteur descripteur réduit **6** est ainsi plus faible que la dimension du vecteur descripteur **5** extrait. La projection **E22** permet de minimiser l'augmentation importante du volume de données (effet dit de « la malédiction de la dimension » ou « curse of dimensionality » selon la terminologie anglo-saxonne) qui isole et éparpille les données ce qui rend le procédé inopérant.

Le traitement **E2** est suivi d'une étape de post-traitement **E3** des vecteurs descripteurs réduits **6.** Le post-traitement **E3** comprend tout d'abord une conversion **E31** de chaque vecteur descripteur réduit **6** en une valeur scalaire **7.** La valeur scalaire **7** représente une vraisemblance d'appartenance du sous-volume **4,** duquel a été extrait le vecteur descripteur réduit **6,** à une classe saine. La conversion **E31** met en œuvre une matrice de covariance **12,** dite saine, obtenue au moyen d'un modèle d'estimation de vraisemblance **11** préalablement appris. La conversion **E31** est obtenue au moyen d'un calcul d'une distance de Mahalanobis prenant en compte la matrice de covariance saine **12.** La distance de Mahalanobis accorde un poids moins important aux composantes les plus dispersées, cela permet de minimiser l'influence des composantes les plus bruitées (celles ayant la plus grande variance). Optionnellement, la distance calculée est normalisée afin d'obtenir des distances comprises dans une plage fixe, par exemple, entre 0 et 1.

Le post-traitement **E3** comprend ensuite une agrégation **E32** des valeurs scalaires **7** par zones de visualisation **3.** L'agrégation **E32** met en œuvre un calcul de moyenne des valeurs scalaires **7** dans chacune des zones de visualisation **3.** Le résultat de ce calcul donne une estimation de l'appartenance **8** à la classe saine pour chaque zone de visualisation **3** du volume tomographique **2.**

Ce post-traitement **E3** permet un contrôle **E4** d'une présence éventuelle de défauts **d** sur la pièce composite tissée **1** dont le volume tomographique **2** a été analysé. Le contrôle **E4** prend en compte les estimations de l'appartenance **8** à la classe saine des zones de visualisation **3** du volume tomographique **2,** obtenues par le post-traitement **E3** afin de déterminer si une des zones de visualisation **3** présente un défaut **d.** Cette étape de contrôle **E4** peut être mise en œuvre de plusieurs manières, par exemples par un inspecteur qualifié. Au cours de cette étape l'inspecteur vérifiera les zones de visualisation **3** du volume tomographique **2** dont l'estimation de l'appartenance **8** à la classe saine est inférieure à un certain seuil fixé. Ce contrôle permet de vérifier la présence effective de défauts d dans ces zones de visualisation **3** et uniquement dans celles-ci, afin de gagner du temps.

La mise en œuvre des étapes de ce procédé de contrôle non-destructif peut être automatisée grâce à l'apprentissage préalable du modèle de détection.

### Apprentissage du modèle de détection

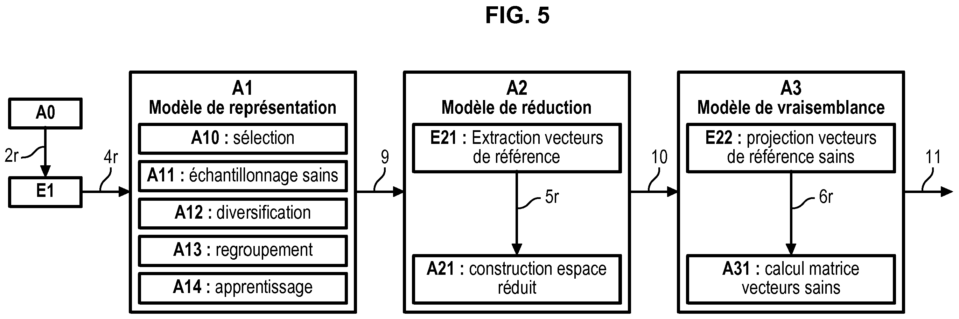

La **figure 5** illustre des étapes d'une mise en œuvre possible de l'apprentissage du modèle de détection. Le modèle de détection utilise trois sous modèles : le modèle de représentation **9,** mis en œuvre au cours de l'extraction E21 précédemment décrite, le modèle de réduction de dimension **10,** mis en œuvre au cours de la projection E22 précédemment décrite, et un modèle d'estimation de vraisemblance **11,** mis en œuvre au cours de la conversion E31 précédemment décrite également.

Afin de mettre en œuvre cet apprentissage, un jeu de données, comprenant un ou plusieurs volumes tomographiques **2r,** dit de référence, sont fournis **A0** à l'unité de calcul. Les volumes tomographiques de référence **2r** sont prétraités (étape **E1)** pour donner des sous-volumes de référence **4r,** de la même manière que pour les volumes tomographiques **2** au cours de la mise en œuvre du procédé de contrôle non-destructif. Le jeu de données, autrement dit l'ensemble des sous-volumes tomographiques de référence **4r,** ainsi fourni (étape **A0**) et prétraité (étape **E1)** compose une base de données pour une création **A1** du modèle de représentation **9.**

La création **A1** du modèle de représentation **9** met en œuvre plusieurs étapes. Cette création **A1** est basée sur une technique d'apprentissage dit supervisé. Le modèle de représentation **9** permet l'extraction de vecteurs descripteurs de référence **5r** pour les sous-volumes de référence **4r.** La création **A1** met tout d'abord en œuvre une sélection **A10** des sous-volumes de référence **4r.** Les sous-volumes de référence **4r** sont annotés par des inspecteurs humains qualifiés qui indiquent les localisations de zones correspondant à des défauts **d.** L'inspecteur attribut à chaque sous-volume de référence **4r** soit une valeur « sans défaut » (« sain ») soit une valeur « défaut » (« anomalie »). La sélection **A10** est suivie d'un tri **A11** qui permet de conserver tous les sous-volumes de référence **4r** de types non-sains et d'échantillonner de manière aléatoire les sous-volumes de référence **4r** de types sains de telle sorte qu'ils soient uniformément répartis dans les zones de visualisations **3** des volumes tomographiques de références **2r.** Ce tri **A11** permet d'améliorer l'équilibre des classes dans la base de données. Selon un mode de mise en œuvre, le ratio entre les sous-volumes de référence **4r** de types sains et ceux de type non-sain dans la base de données est établi à trois pour un.

La création **A1** du modèle de représentation **9** comprend, en outre, une étape de diversification **A12** des sous-volume de référence **4r** sélectionnés de manière à augmenter la variété des sous-volumes de référence **4r** de types non-sains. La diversification **A12** met en œuvre une transformation géométrique de plusieurs sous-volumes de référence **4r** qui induit une multiplication de ces sous-volumes de références **4r.** Les transformations géométriques appliquées sont, par exemple, des effets miroirs, des rotations aléatoires, des déformations affines, ou des découpes aléatoires... Seuls les effets miroirs sont appliqués à tous les sous-volumes de référence **4r** de types sains, tandis que toutes les transformations géométriques le sont aux sous-volumes de référence **4r** de types non-sains. Ces transformations permettent de créer artificiellement de la variété au sein des sous-volumes de référence **4r** et d'augmenter davantage la représentation et la variété des sous-volumes de référence **4r** de types non-sains. Cette étape permet, entre autres, de pallier à la faible quantité de sous-volumes de référence **4r** annotées.

La diversification **A12** précède une étape de regroupement **A13** des différents sous-volumes de référence **4r,** ainsi multipliés, en regroupant des sous-volumes de référence **4r** de types sains et de types non-sains dans des sous-ensembles (ou « batch » selon la terminologie anglo-saxonne). Les sous-volumes **4** de types sains sont donc nécessairement regroupés dans un sous-ensemble comprenant des sous-volumes de référence **4r** de types non-sains. Le ratio de sous-volumes de référence **4r** de types sains et de types non-sains dans les différents sous-ensembles est préférentiellement constant. Ainsi le modèle de représentation **9** ne sera pas faussé par une sur-représentation locale de sous-volumes **4** de types sains.

Ces étapes sont suivies d'un apprentissage **A14** d'un réseau de neurones. Le réseau de neurones reçoit en entrée les sous-volumes de référence **4r** regroupés en sous-ensembles et met en œuvre un apprentissage afin de fournir le modèle de représentation **9.** L'apprentissage **A14** mis en œuvre est préférentiellement un apprentissage profond. L'apprentissage **A14** met en œuvre une technique semi-supervisé avec une approche dite Mean Teacher (Tarvainen, 2017) (Wang, 2021). Une telle technique de garantir un apprentissage efficace malgré le manque de sous-volumes de référence **4r.** Cette démarche utilise deux réseaux nommés « Teacher » et « Student ». Ici, les poids du réseau Teacher sont définis comme une moyenne mobile exponentielle (EMA) des poids du réseau Student. C'est-à-dire qu'après chaque étape de « formation », les paramètres du réseau Teacher sont légèrement mis à jour à partir des paramètres du réseau Student. Ce réseau Student est optimisé selon la fonction d'erreur focale choisie et combiné à une fonction de perte de cohérence (ou « Consistency Loss » selon la terminologie anglo-saxonne) qui pénalise lorsque les réponses du réseau Student s'éloignent des réponses du réseau Teacher. L'approche Mean Teacher va particulièrement être efficace pour favoriser la conservation et la distillation de l'information dans l'ensemble du réseau. Une fois l'apprentissage terminé les poids du réseau Student sont fixés pour son utilisation en mode contrôle (ou inférence) dans le procédé de contrôle non destructif. L'apprentissage **A14** met en œuvre une fonction d'erreur focal et un algorithme d'optimisation afin d'en améliorer les performances.

Selon une mise en œuvre, le réseau de neurone comprend une architecture de type : ResNet10 (He), ResNet18 (He), ResNet50, DenseNet121 ou ViT et préférentiellement ResNet50.

Selon une mise en œuvre alternative, l'apprentissage **A14** met en œuvre une technique supervisée.

L'apprentissage **A14** est couplé à l'algorithme d'optimisation qui favorise une convergence lente afin de favoriser la distillation de l'information et la stabilisation des paramètres du réseau de neurones. Dans la démarche proposée ici, au lieu de garder tous les poids du réseau de neurones, la dernière couche de classification est éliminée, de sorte que la sortie du modèle corresponde à la sortie de la couche avant-dernière (ou « average pooling » selon la terminologie anglo-saxonne), qui fait office d'extracteur de descripteur.

La fonction d'erreur focale mise en œuvre est préférentiellement une fonction de type « Focal Loss » (Lin., 2017) ou de type entropie croisée qui sont particulièrement adaptée lorsque le jeu de données est mal équilibré. Un exemple est illustré par les présentes équations : $focal loss \left(x, y\right) = - {α}_{c} {\left(1-{p}_{c}\right)}^{γ} log \left({p}_{c}\right) ⋅ 1 \left\{y\neq ignored label\right\} , où p = \frac{exp \left({x}_{y}\right)}{{\sum }_{c = 1}^{C} exp \left({x}_{C}\right)}$ $entropie crois é e \left(x, y\right) = - {w}_{y} log \left(\frac{exp \left({x}_{y}\right)}{{\sum }_{c = 1}^{C} exp \left({x}_{C}\right)}\right) ⋅ 1 \left\{y\neq ignored label\right\}$

Avec, p correspondant à la sortie du réseau de neurone et α à un coefficient de pondération fixé à 0,25 et γ à facteur de pénalisation (ou modulation) fonction des difficultés de prédiction d'appartenance à un des types.

L'algorithme d'optimisation, est par exemple l'algorithme Adam (Kingma, 2014) ou SGD ou RMSProp ...

Ainsi, au lieu d'avoir un modèle qui fournit une réponse binaire pour chaque sou-volume de référence **4** donné, il fournit un vecteur descripteur **5,** par exemple de longueur 2048. L'apprentissage **A14** du réseau de neurone permet donc d'obtenir un modèle qui est le modèle de représentation **9** mis en œuvre dans le procédé de contrôle non-destructif.

L'apprentissage du modèle de détection met ensuite en œuvre une création **A2** d'un modèle de réduction de dimension **10.** Cette création **A2** permet la projection **E22** des vecteurs descripteurs **5** de manière à réduire leur dimension et de ne conserver que les informations les plus pertinentes vis-à-vis de la détection de défauts **d.** La création A2 du modèle de réduction de dimension **10** met tout d'abord en œuvre l'extraction **E21** de vecteurs descripteurs de référence **5r** des sous-volumes de référence **4r,** et ce indifféremment de leur annotation. La création **A2** comprend, en outre, la mise en œuvre d'un calcul de construction **A21** d'un espace réduit. Le calcul **A21** réalise l'orthogonalisation d'une matrice de covariance des vecteurs descripteurs de référence **5r** extraits en utilisant la méthode d'analyse en composantes principales précédemment décrite. Ainsi seulement un certain nombre de vecteurs propres parmi leur totalité, issus de l'orthogonalisation, est sélectionné, afin de réduire la dimension de ces vecteurs descripteur de référence **5r** extraits. Par exemple, il est possible de conserver seulement les 50 premiers des 2048 vecteurs propres de chaque vecteur descripteur de référence **5r** extraits, qui permettent d'exprimer 99% de la variance totale. Le modèle de réduction de dimension **10** permet donc d'obtenir un espace réduit pour condenser les vecteurs descripteurs **5** extraits.

Enfin une création **A3** d'un modèle d'estimation de vraisemblance **11** est mise en œuvre. Elle est propre à permettre la conversion **E31** du vecteur descripteur réduit **6** de chaque sous-volume **4** en valeur scalaire **7.** Pour cela, la création **A3** met en œuvre la projection **E22,** décrite ci-dessus, des vecteurs descripteurs de référence **5r** extraits annotés de types sains, dans le modèle de réduction de dimensions **10** pour obtenir des vecteurs descripteurs de référence réduits **6r** de type sain. Ensuite, il réalise un calcul **A31** de la matrice de covariance saine **12** de ces vecteurs descripteurs de référence réduits **6r** de type sain. Cette matrice de covariance saine **12** étant le modèle d'estimation de vraisemblance **11** permettant la conversion **E31.** En d'autres termes, la matrice de covariance saine **12** représente un domaine des sous-volumes **4** de la classe saine.

### Evaluation du procédé

Afin de pouvoir évaluer le comportement général de l'approche pour une pluralité de scénarios, la métrique de l'aire sous la courbe (ou « area under curve », soit AUC, selon la terminologie anglo-saxonne) d'une courbe caractéristique de fonctionnement du récepteur (ou « receiver operating characteristic », ou ROC, selon la terminologie anglo-saxonne) est utilisée. La courbe ROC est un outil souvent utilisé pour les cas de détection binaires et qui permets de caractériser l'évolution du taux de bonne détection en fonction du taux de fausses alarmes. Cette mesure permet de rendre compte du pouvoir prédictif du procédé, puisque cela met en évidence sa qualité à discriminer un échantillon anormal.

Dans le cas d'un estimateur parfait, l'AUC de la courbe ROC serait proche de l'unité, tandis que dans le cas d'un estimateur « aléatoire », elle serait de 0,5.

De plus, la courbe ROC et son AUC permettent de rendre compte directement de critères industriels comme celui de minimiser au mieux la quantité de fausses alarmes tout en maximisant la bonne localisation des indications. En d'autres termes, l'objectif est d'éviter de fournir aux inspecteurs des zones saines à examiner, le tout en lui fournissant l'ensemble complet des zones douteuses.

Ainsi, la démarche proposée a été mise en place à pour un ensemble de 31 aubes de soufflante où 23 de ces pièces contenaient une anomalie de tissage, tel qu'annotées par un inspecteur qualifié avec le masque binaire associé. Ces dernières ont été utilisées pour évaluer les performances des modèles lors de l'apprentissage.

Par la suite, une base de 28 pièces dont 16 avec anomalies, aussi annotées par des inspecteurs qualifiés a été constituée. Ces pièces ont été traitées par le procédé proposée en mode contrôle (ou « inférence ») et les résultats ont été utilisés pour construire des courbes ROC.

Les résultats obtenus montrent un très bon comportement de la démarche proposée, laquelle est capable d'atteindre une AUC de 96.06% pour l'identification des défauts **d.** Ceci est atteint en respectant des contraintes industrielles de taux de bonne détection supérieur à 80% et taux de fausse alarme inférieur à 30% pour plusieurs points de fonctionnement de la méthode. De plus, la méthode est capable d'obtenir une AUC très élevée de 99.46% pour des types de défauts **d** qui n'étaient pas présent dans la base de données initialement fournie pour l'apprentissage (les 31 premières aubes). Ainsi, ces résultats démontrent aussi que la démarche proposée est capable de généraliser pour nouveaux types de défauts **d.**

### Intégration dans un processus d'analyse de qualité

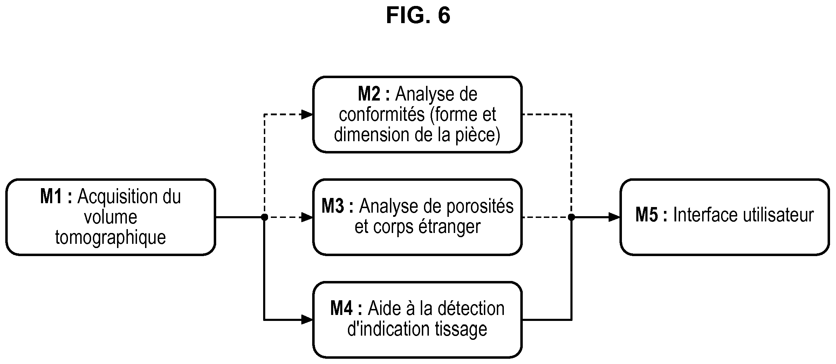

Le procédé **M4** de contrôle non destructif de pièce composite tissée **1** peut être mis en œuvre dans un processus d'analyse de qualité. Le processus d'analyse qualité comprend plusieurs étapes dont certaines sont schématisées par la **figure 6****.** Une étape préliminaire d'acquisition **M1** est mise en œuvre à l'aide d'un système d'imagerie, comme un tomographe. Cette étape permet d'obtenir une image tridimensionnelle de la pièce **1,** autrement dit un volume tomographique **2.** Ce volume tomographique **2** est transmis à un module mettant en œuvre le procédé de contrôle non destructif tel que précédemment décrit.

Le processus d'analyse qualité peut, en outre comprendre une analyse de conformité **M2** du volume tomographique **2** à la forme et la dimension de la pièce fabriquée ainsi qu'une analyse de porosité **M3** et de présence de corps étrangers dans le volume de cette pièce. L'analyse **M3** peut par exemple être mise en œuvre au moyen d'une macro-programme afin de permettre de calculer automatiquement le taux de porosité de chacune des pièces, comme présenté par exemple dans le document FR 3 046 844 A1.

Une unité de calcul est prévue pour effectuer des traitements informatiques. L'unité de calcul met en œuvre le processus d'analyse de qualité et plus particulièrement les étapes d'acquisition **M1,** d'analyse de conformité **M2,** d'analyse de porosité **M3** et de présence de corps étrangers ainsi que le procédé de contrôle **M4.** A cette fin l'unité de calcul comprend plusieurs modules. L'unité de calcul stocke le modèle de détection, précédemment décrit et mis en œuvre dans le procédé de contrôle **M4.** L'unité de calcul comprend des moyens de traitement de données et une mémoire. En outre, une telle unité peut comprendre un module propre à exécuter une étape d'affichage **M5** des résultats des analyses et de contrôle.

## Revendications

1. Procédé mise en œuvre par ordinateur
de contrôle non destructif d'une pièce composite tissée (1), dans lequel un volume tomographique (2) préalablement acquis et reconstruit est divisé en une pluralité de zones de visualisations (3), le procédé comprenant les étapes suivantes de :
- pré-traitement (E1) dudit volume tomographique (2), comprenant un découpage (E13) des zones de visualisations (3) du volume tomographique (2) en des sous-volumes (4) tridimensionnels ;
- traitement (E2) de chaque sous-volume (4), ledit traitement comprenant, pour chaque sous-volume (4),
d'une part, une extraction (E21) d'un vecteur descripteur (5), ladite extraction mettant en œuvre une analyse d'image au moyen d'un modèle de représentation (9) préalablement appris, ladite analyse d'image décomposant chaque sous-volume (4) en une pondération appliquée à une pluralité de descripteurs dudit modèle de représentation (9), le vecteur descripteur (5) étant composé des poids de cette décomposition, et
d'autre part, une projection (E22) du vecteur descripteur (5) extrait dans un modèle de réduction de dimension (10) préalablement appris, de manière à condenser le vecteur descripteur (5) en un vecteur descripteur réduit (6), de dimension plus faible ;
- post-traitement (E3) des vecteurs descripteurs réduits (6), comprenant :
d'une part une conversion (E31), au moyen d'un modèle d'estimation de vraisemblance (11) préalablement appris, du vecteur descripteur réduit (6) de chaque sous-volume (4) en une valeur scalaire (7), ladite valeur scalaire (7) représentant une vraisemblance d'appartenance du sous-volume à une classe saine, et
d'autre part une agrégation (E32) par calcul de moyenne des valeurs scalaires (7) dans chacune des zones de visualisation (3), le résultat de ce calcul étant une estimation de l'appartenance (8) à la classe saine pour chaque zone de visualisation (3) du volume tomographique (2) ; et
- contrôle (E4) d'une présence de défauts (d) sur la pièce composite tissée (1), en fonction des estimations de l'appartenance (8) à la classe saine des zones de visualisation (3) du volume tomographique (2) ainsi obtenues.

2. Procédé selon la revendication 1, comprenant une étape de création (A1) du modèle de représentation (9), propre à permettre l'extraction (E21) de vecteurs descripteurs (5), et comprenant des étapes de :
- sélection (A10) de sous-volumes de référence (4r), formant un volume tomographique de référence (2r), annotés comme étant d'un type sain ou d'un type défectueux,
- diversification (A12) des sous-volumes de référence (4r), ladite diversification (A12) mettant en œuvre une multiplication et une transformation géométrique d'un ou plusieurs sous-volumes de référence (4r), et
- apprentissage (A14) d'un réseau de neurones au moyen des sous-volumes de référence (4r) diversifiés pour obtenir un modèle de représentation (9) des sous-volumes de référence (4r), l'apprentissage mettant en œuvre une fonction d'erreur et un algorithme d'optimisation.

3. Procédé selon la revendication 2, comprenant une étape de création (A2) du modèle de réduction de dimension (10) propre à permettre la projection (E22) de vecteurs descripteurs (5) extraits, et comprenant la mise en œuvre :
- de l'extraction (E21) de vecteurs descripteurs de référence (5r) des sous-volumes de référence (4r), au moyen du modèle de représentation (9),
- d'un calcul de construction (A21) d'un espace réduit, ledit calcul de construction (A21) mettant en œuvre une orthogonalisation d'une première matrice de covariance des vecteurs descripteurs de référence (5r) extraits.

4. Procédé selon la revendication 3, comprenant une étape de création (A3) du modèle d'estimation de vraisemblance (11), propre à permettre la conversion (E31) de vecteurs descripteurs réduits (6), ledit apprentissage mettant en œuvre :
- la projection (E22), dans l'espace réduit, des vecteurs descripteurs de référence (5r) extraits annotés comme étant type sain, de manière à les condenser en des vecteurs descripteurs de référence réduits (6r) de type sain, et
- un calcul (A31) d'une seconde matrice de covariance (12) des vecteurs descripteurs de référence réduits (6r) de type sain représentant un domaine de la classe saine.

5. Procédé selon l'une quelconque des revendications 1 à 4, dans lequel la projection (E22) du vecteur descripteur (5) en un vecteur descripteur réduit (6) met en œuvre une méthode d'analyse en composantes principales (PCA).

6. Procédé selon l'une quelconque des revendications 2 à 4, dans lequel la création (A1) du modèle de représentation (9) comprend un regroupement (A13) des sous-volumes de référence (4r) obtenus par la diversification (A12) en des sous-ensembles comprenant chacun des sous-volumes de référence (4r) de types sain et défectueux.

7. Procédé selon l'une quelconque des revendications 1 à 6, dans lequel le pré-traitement (E1) comprend une étape de normalisation globale des niveaux de gris (E12) du volume tomographique (3) adapté pour en améliorer le contraste.

8. Procédé selon l'une quelconque des revendications 1 à 7, dans lequel le découpage (E13) des zones de visualisations (3) met en œuvre un recouvrement entre chaque paire de sous-volume (4) adjacent, d'une même zone de visualisation (3) uniquement, afin de minimiser le taux de fuite.

9. Procédé selon l'une quelconque des revendications 1 à 8, dans lequel le pré-traitement (E1) comprend une étape de correction locale (E14) du contraste mettant en œuvre une normalisation d'histogramme de chaque sous-volume (4) découpé.

10. Procédé selon l'une quelconque des revendications 1 à 9, dans lequel le contrôle (E4) d'une présence de défauts (d) sur la pièce composite tissée (1) met en œuvre une comparaison des estimations de l'appartenance (8) à la classe saine à un seuil prédéfini.

11. Produit programme d'ordinateur, comprenant des instructions de code pour exécuter le procédé de contrôle non destructif selon l'une quelconque des revendications 1 à 10, lorsqu'il est exécuté sur des moyens de traitement d'une unité de calcul.

## Patentansprüche

1. Computergesteuertes Verfahren zur zerstörungsfreien Prüfung eines gewebten Verbundwerkstoffteils (1), bei dem ein zuvor erfasstes und rekonstruiertes tomographisches Volumen (2) in eine Vielzahl von Visualisierungsbereichen (3) unterteilt wird, wobei das Verfahren die folgenden Schritte umfasst:
- Vorverarbeiten (E1) des tomographischen Volumens (2), umfassend ein Schneiden (E13) der Visualisierungsbereiche (3) des tomographischen Volumens (2) in dreidimensionale Teilvolumina (4);
- Verarbeiten (E2) jedes Teilvolumens (4), wobei die Verarbeitung für jedes Teilvolumen (4) umfasst
einerseits eine Extraktion (E21) eines Deskriptorvektors (5), wobei die Extraktion eine Bildanalyse mittels eines zuvor gelernten Repräsentationsmodells (9) durchführt, wobei die Bildanalyse jedes Teilvolumen (4) in eine Gewichtung zerlegt, die auf eine Vielzahl von Deskriptoren des Repräsentationsmodells (9) angewendet wird, wobei der Deskriptorvektor (5) aus den Gewichten dieser Zerlegung zusammengesetzt ist, und
andererseits eine Projektion (E22) des extrahierten Deskriptorvektors (5) in ein zuvor gelerntes Dimensionsreduktionsmodell (10), um den Deskriptorvektor (5) in einen reduzierten Deskriptorvektor (6) mit niedrigerer Dimension zu kondensieren;
- Nachbearbeiten (E3) der reduzierten Deskriptorvektoren (6), umfassend:
einerseits eine Umwandlung (E31) des reduzierten Deskriptorvektors (6) jedes Teilvolumens (4) in einen Skalarwert (7) mittels eines zuvor gelernten Wahrscheinlichkeits-Schätzmodells (11), wobei der Skalarwert (7) eine Zugehörigkeits-Wahrscheinlichkeit des Teilvolumens zu einer fehlerfreien Klasse darstellt; und
andererseits eine Aggregation (E32) durch Berechnung des Mittelwerts der Skalarwerte (7) in jedem der Visualisierungsbereiche (3), wobei das Ergebnis dieser Berechnung eine Schätzung der Zugehörigkeit (8) zu der fehlerfreien Klasse für jeden Visualisierungsbereich (3) des tomographischen Volumens (2) ist; und
- Kontrollieren (E4) auf das Vorhandensein von Defekten (d) an dem gewebten Verbundwerkstoffteil (1) in Abhängigkeit von den so erhaltenen Schätzungen der Zugehörigkeit (8) zu der fehlerfreien Klasse der Visualisierungsbereiche (3) des tomographischen Volumens (2).

2. Verfahren nach Anspruch 1, das einen Schritt der Erstellung (A1) des Repräsentationsmodells (9) umfasst, das geeignet ist, die Extraktion (E21) von Deskriptorvektoren (5) zu ermöglichen, und die folgenden Schritte umfasst:
- Auswählen (A10) von Referenz-Teilvolumina (4r), die ein Referenz-Tomographievolumen (2r) bilden, die als fehlerfrei oder fehlerhaft gekennzeichnet sind,
- Diversifizieren (A12) der Referenz-Teilvolumina (4r), wobei die Diversifizierung (A12) eine Multiplikation und eine geometrische Transformation eines oder mehrerer Referenz-Teilvolumina (4r) beinhaltet, und
- Trainieren (A14) eines neuronalen Netzes mittels der diversifizierten Referenz-Teilvolumina (4r), um ein Repräsentationsmodell (9) der Referenz-Teilvolumina (4r) zu erhalten, wobei das Training eine Fehlerfunktion und einen Optimierungsalgorithmus beinhaltet.

3. Verfahren nach Anspruch 2, das einen Schritt der Erstellung (A2) des Dimensionsreduktionsmodells (10) umfasst, das geeignet ist, die Projektion (E22) extrahierter Deskriptorvektoren (5) zu ermöglichen, und die Durchführung beinhaltet:
- die Extraktion (E21) von Referenz-Deskriptorvektoren (5r) der Referenz-Teilvolumina (4r) mittels des Repräsentationsmodells (9),
- eine Konstruktionsberechnung (A21) eines reduzierten Raums, wobei die Konstruktionsberechnung (A21) eine Orthogonalisierung einer ersten Kovarianzmatrix der extrahierten Referenz-Deskriptorvektoren (5r) beinhaltet.

4. Verfahren nach Anspruch 3, das einen Schritt der Erstellung (A3) des Wahrscheinlichkeits-Schätzmodells (11) umfasst, das geeignet ist, die Umwandlung (E31) reduzierter Deskriptorvektoren (6) zu ermöglichen, wobei das Training beinhaltet:
- die Projektion (E22) der extrahierten Referenz-Deskriptorvektoren (5r), die als fehlerfrei gekennzeichnet sind, in den reduzierten Raum, um sie in reduzierte fehlerfreie Referenz-Deskriptorvektoren (6r) zu kondensieren, und
- eine Berechnung (A31) einer zweiten Kovarianzmatrix (12) der reduzierten fehlerfreien Referenz-Deskriptorvektoren (6r), die einen Bereich der fehlerfreien Klasse darstellt.

5. Verfahren nach einem der Ansprüche 1 bis 4, wobei die Projektion (E22) des Deskriptorvektors (5) auf einen reduzierten Deskriptorvektor (6) eine Hauptkomponentenanalyse (PCA) beinhaltet.

6. Verfahren nach einem der Ansprüche 2 bis 4, wobei die Erzeugung (A1) des Repräsentationsmodells (9) eine Gruppierung (A13) der durch die Diversifizierung (A12) erhaltenen Referenz-Teilvolumina (4r) in Teilmengen umfasst, die jeweils fehlerfreie und fehlerhafte Referenz-Teilvolumina (4r) umfassen.

7. Verfahren nach einem der Ansprüche 1 bis 6, wobei die Vorverarbeitung (E1) einen globalen Normalisierungsschritt der Graustufen (E12) des tomographischen Volumens (3) umfasst, der zur Verbesserung seines Kontrasts geeignet ist.

8. Verfahren nach einem der Ansprüche 1 bis 7, wobei das Schneiden (E13) der Visualisierungsbereiche (3) eine Überlappung zwischen jedem Paar benachbarter Teilvolumina (4) nur desselben Visualisierungsbereichs (3) beinhaltet, um die Leckrate zu minimieren.

9. Verfahren nach einem der Ansprüche 1 bis 8, wobei die Vorverarbeitung (E1) einen lokalen Kontrastkorrekturschritt (E14) umfasst, der eine Histogrammnormalisierung jedes geschnittenen Teilvolumens (4) beinhaltet.

10. Verfahren nach einem der Ansprüche 1 bis 9, wobei die Kontrolle (E4) eines Vorhandenseins von Fehlern (d) auf dem gewebten Verbundwerkstoffteil (1) einen Vergleich der Schätzungen der Zugehörigkeit (8) zu der fehlerfreien Klasse mit einem vordefinierten Schwellenwert beinhaltet.

11. Computerprogrammprodukt, umfassend Codeanweisungen zur Ausführung des zerstörungsfreien Prüfverfahrens nach einem der Ansprüche 1 bis 10, wenn es auf Verarbeitungsmitteln einer Recheneinheit ausgeführt wird.

## Claims

1. A method for the non-destructive testing of a woven composite part (1), in which a previously acquired and reconstructed tomographic volume (2) is divided into a plurality of visualization zones (3), the method comprising the following steps:
- pre-processing (E1) said tomographic volume (2), comprising dividing
- (E13) of the visualization zones (3) of the tomographic volume (2) into three-dimensional sub-volumes (4);
- processing (E2) each sub-volume (4), said processing comprising, for each sub-volume (4),
on the one hand, an extraction (E21) of a descriptor vector (5), said extraction implementing an image analysis by means of a previously trained representation model (9), said image analysis decomposing each sub-volume (4) into a weighting applied to a plurality of descriptors of said representation model (9), the descriptor vector (5) being composed of the weights of this decomposition, and
on the other hand, a projection (E22) of the extracted descriptor vector (5) into a previously trained dimension reduction model (10), so as to condense the descriptor vector (5) into a reduced descriptor vector (6), of lower dimension;
- post-processing (E3) of the reduced descriptor vectors (6), comprising:
on the one hand a conversion (E31), by means of a previously trained likelihood estimation model (11), of the reduced descriptor vector (6) of each sub-volume (4) into a scalar value (7), said scalar value (7) representing a likelihood of the sub-volume belonging to a healthy class, and
on the other hand an aggregation (E32) by calculating the average of the scalar values (7) in each of the visualization zones (3), the result of this calculation being an estimate of the membership (8) of the healthy class for each visualization zone (3) of the tomographic volume (2); and
- checking (E4) for the presence of defects (d) in the woven composite part (1), according to the estimates of the membership (8) of the healthy class of the thus-obtained visualization zones (3) of the tomographic volume (2).

2. The method according to claim 1, comprising a step of creating (A1) the representation model (9), capable of allowing the extraction (E21) of descriptor vectors (5), and comprising steps of:
- selecting (A10) reference sub-volumes (4r), forming a reference tomographic volume (2r), annotated as being of a healthy type or a defective type,
- diversifying (A12) the reference sub-volumes (4r), said diversification (A12) implementing a multiplication and a geometric transformation of one or more reference sub-volumes (4r), and
- training (A14) a neural network using the diversified reference sub-volumes (4r) to obtain a representation model (9) of the reference sub-volumes (4r), the training implementing an error function and an optimization algorithm.

3. The method according to claim 2, comprising a step of creating (A2) the dimension reduction model (10) capable of allowing the projection (E22) of extracted descriptor vectors (5), and comprising the implementation:
- of the extraction (E21) of reference descriptor vectors (5r) from the reference sub-volumes (4r), by means of the representation model (9),
- of a construction calculation (A21) of a reduced space, said construction calculation (A21) implementing an orthogonalization of a first covariance matrix of the extracted reference descriptor vectors (5r).

4. The method according to claim 3, comprising a step of creating (A3) the likelihood estimation model (11), capable of allowing the conversion (E31) of reduced descriptor vectors (6), said training implementing:
- the projection (E22), in the reduced space, of the extracted reference descriptor vectors (5r) annotated as being of healthy type, so as to condense them into reduced reference descriptor vectors (6r) of healthy type, and
- a calculation (A31) of a second covariance matrix (12) of the reduced reference descriptor vectors (6r) of healthy type representing a domain of the healthy class.

5. The method according to any one of claims 1 to 4, wherein the projection (E22) of the descriptor vector (5) into a reduced descriptor vector (6) implements a principal component analysis (PCA) method.

6. The method according to any one of claims 2 to 4, wherein the creation (A1) of the representation model (9) comprises a grouping (A13) of the reference sub-volumes (4r) obtained by diversification (A12) into subsets each comprising reference sub-volumes (4r) of healthy and defective types.

7. The method according to any one of claims 1 to 6, wherein the pre-processing (E1) comprises a step of global normalization of the gray levels (E12) of the adapted tomographic volume (3) to improve the contrast.

8. The method according to any one of claims 1 to 7, wherein the dividing (E13) of the visualization zones (3) implements an overlap between each pair of adjacent sub-volumes (4), of the same visualization zone (3) only, in order to minimize the leakage rate.

9. The method according to any one of claims 1 to 8, wherein the pre-processing (E1) comprises a step of local correction (E14) of the contrast implementing a histogram normalization of each cut sub-volume (4).

10. The method according to any one of claims 1 to 9, wherein the testing (E4) of a presence of defects (d) on the woven composite part (1) implements a comparison of the estimates of the membership (8) of the healthy class with a predefined threshold.

11. A computer program product, comprising code instructions for executing the non-destructive testing method according to any one of claims 1 to 10, when executed on processing means of a calculation unit.
